# EUROPÄISCHE PATENTSCHRIFT

(11) **EP 0 591 594 B1**
(45) Veröffentlichungstag und Bekanntmachung des Hinweises auf die Patenterteilung: **02.05.1997**
(21) Anmeldenummer: 92810761.4
(22) Anmeldetag: 07.10.1992
(51) Int. Cl.: A61F 2/34

(54) **Acetabulum-Pfannenstützring**
Acetabular cup supporting ring
Anneau d'appui pour coque acétubulaire

(43) Veröffentlichungstag der Anmeldung: 13.04.1994
(73) Patentinhaber: OSTEO AG, 2545 Selzach (CH)
(72) Erfinder: Zichner, Ludwig, Prof. Dr. med., W-6078 Neu-Isenburg (DE); Wahl, Thomas, CH-2543 Lengnau (CH); Leu, Beat, CH-6052 Hergiswil (CH)
(74) Vertreter: AMMANN PATENTANWAELTE AG BERN

(56) Entgegenhaltungen:
- EP-A- 0 123 514
- EP-A- 0 313 773
- EP-A- 0 486 403
- DE-A- 4 102 510
- FR-A- 2 233 976
- FR-A- 2 660 546
- US-A- 3 863 273

## Beschreibung

Acetabulum-Pfannenstützringe sind in verschiedenen Ausführungsformen bekannt und werden hauptsächlich bei Hüftgelenks-Revisionsoperationen verwendet, die mit Problemen behaftet sind. Solche Probleme entstehen insbesondere durch die knochenresorbierende Wirkung des Knochenzementes, die meist grossvolumige Knochendefekte verursacht. Dann ist eine Verankerung eines zementlosen standardisierten Acetabulumimplantates meist äusserst schwierig. Auch bei Dysplasie-Hüften ist eine Verankerung der Hüftpfanne sehr problematisch.

Aus der EP-A-0 123 514 ist eine Acetabulum-Pfannenbefestigungsvorrichtung gemäss Oberbegriff von Patentanspruch 1 bekannt, die als Befestigungselement eine am Boden geschlossene Pfanne aufweist und, da sie einzementierbar ist, nur eine Befestigungslasche aufweist. Zum Sichern der im Befestigungselement schwenkbaren Pfannenschale sind lediglich zwei Schrauben vorgesehen. Die Verwendung einer am Boden geschlossenen Pfanne bedingt eine relativ grosse Bauhöhe und verstellt die freie Sicht auf den Acetabulum-Boden, während die Verbindung mittels Schrauben die Montage erschwert und keine grosse Sicherheit bietet.

Ferner werden weitere zum Stand der Technik gehörende Stützimplantate mit Knochenschrauben im Acetabulum befestigt und darin die Hüftpfanne mit Knochenzement einzementiert. Eine solche Anordnung weist jedoch wiederum die bekannten Nachteile der Verwendung von Knochenzement auf, insbesondere knochenresorbierende Wirkung und ungenügende Dauerschwingfestigkeit, die zu Implantatlockerungen führen. Neben der Notwendigkeit, Knochenzement zur Verankerung der Pfanne zu verwenden, weisen diese vorbekannten Pfannenstützringe den weiteren Nachteil auf, dass sie nur in einem beschränkten Umfang in die genau richtige Lage im Becken einsetzbar sind.

Es ist von diesem Stand der Technik ausgehend Aufgabe der vorliegenden Erfindung, eine Acetabulum-Pfannenbefestigungsvorrichtung anzugeben, die ein zementloses Implantieren gewährleistet, eine genaue Ausrichtung der Pfanne im Becken und somit auch bezüglich des Stieles ermöglicht und deren Pfannenschale schnell montiert und gut gesichert ist. Eine solche Vorrichtung ist in den Ansprüchen definiert. Der Pfannenstützring gemäss Patentanspruch 1 weist vier Elemente auf, wobei der Stützring ohne Knochenzement im Becken befestigt wird und darin eine Pfannenschale in einem grossen Winkelbereich justiert eingelegt werden kann und durch einen Sicherungsring ohne Knochenzement gesichert wird. In diese optimal ausgerichtete Pfannenschale ist dann ein Einsatz, in der Regel aus Kunststoff, zur Aufnahme der Gelenkkugel eingelegt. Einerseits wird zur Verankerung des Pfannenstützringes kein Knochenzement verwendet und andererseits erlaubt der modulare Aufbau ein ganz genaues Ausrichten der Pfannenschale.

Die Erfindung wird im folgenden anhand einer Zeichnung eines Ausführungsbeispieles näher erläutert.
Fig. 1 ist eine schematische Explosionszeichnung der vier Teile des erfindungsgemässen Pfannenstützringes,
Fig. 2 zeigt eine Seitenansicht des zusammengesetzten Pfannenstützringes von Fig. 1,
Fig. 3 ist ein Schnitt der Darstellung von Fig. 2,
Fig. 4 zeigt den Einsatz von unten, und
Fig. 5 zeigt im Schnitt den in der Pfannenschale eingesetzten Einsatz.

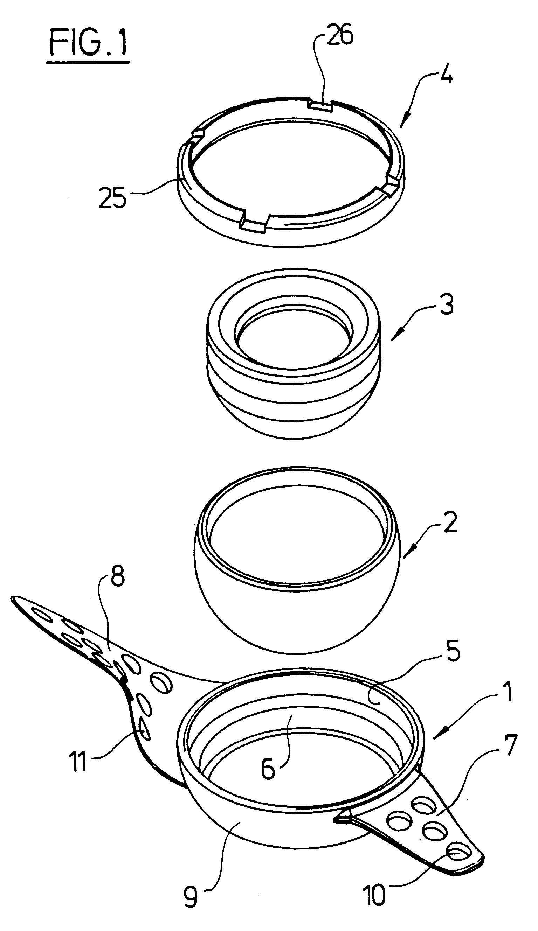

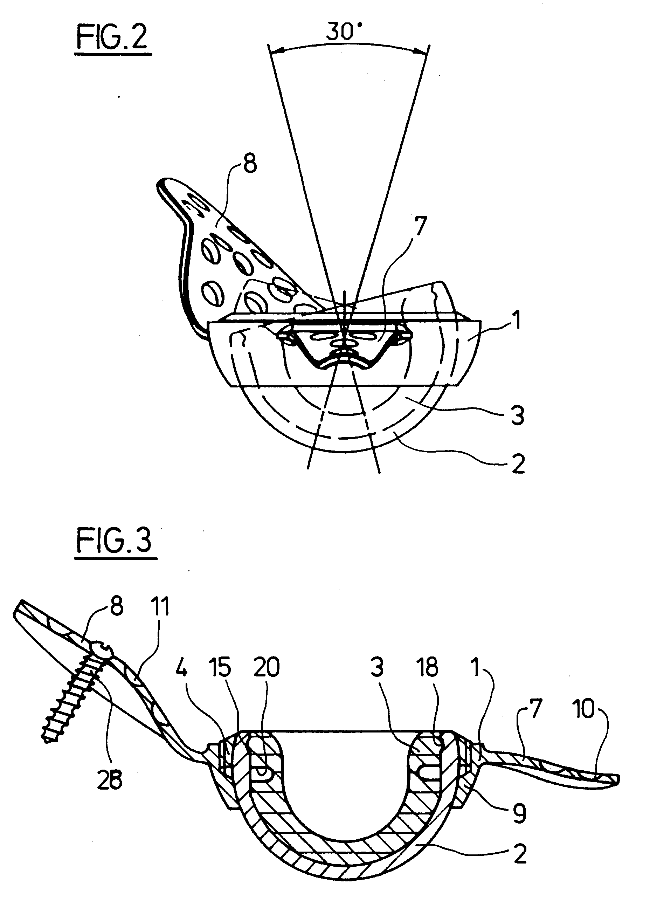

Fig. 1 zeigt die vier Elemente des Pfannenstützringes, von unten nach oben gesehen, den Stützring 1, die Pfannenschale 2, die Einlage 3 und einen als Sicherungselement dienender Sicherungsring 4, wobei die Einlage aus Kunststoff, vorzugsweise Polyethylen oder mindestens teilweise aus Metall besteht, und die anderen Teile aus Metall, z.B. Titan oder eine Titanlegierung oder eine Chrom-Kobalt-Molybdän-Legierung oder auch aus Kunststoff bestehen. Wie am besten aus den Fig. 1 und 3 ersehen werden kann, weist der Stützring ein zylindrisches Teil 5 mit Innengewinde zur Aufnahme des Sicherungsringes und daran anschliessend eine kugelförmige, konzentrische Ausformung 6 zur Aufnahme der Pfannenschale 2 auf. Aussen ist der Stützring mit einer kleinen Lasche 7 und einer grossen Lasche 8 versehen. Die mit dem Knochen in Berührung kommenden Oberflächen aller Metallteile, so auch die Oberfläche 9 des Stützrings, können behandelt sein, um das Anwachsen des Knochens zu verbessern. Beispielsweise kann die Oberfläche durch Glasstrahlen vergrössert sein oder mit einer Hydroxylapatit- oder ähnlich bioaktiven Schicht, z. B. auch ein Calziumphosphatglas versehen sein, die auch aufgesputtert sein kann. Dadurch wird eine Langzeitstabilität durch An- bzw. Einwachsen des Knochens an bzw. in das Implantat erzielt.

Die primäre Stabilität wird durch die beiden Laschen 7 und 8 erzielt. Die kleinere Lasche 7 ist anatomisch geformt, spitz zulaufend und mit Bohrungen 10 versehen, in die der Knochen einwachsen kann und eine höhere Haltefestigkeit bewirkt. Die kleinere Lasche wird in der Regel im Schambein eingeschlagen. Ist das Einschlagen bzw. die Verankerung des Ringes durch ungenügende Knochenfestigkeit im Schambeinbereich nicht möglich, kann der Stützring durch die Löcher 10 mittels Schrauben befestigt werden. Die Ausbildung der Schraubenlöcher ermöglicht nicht nur, die Schraube in der Bohrungsachse zu implantieren, sondern auch diese in einem gewissen Bereich zu schwenken. Die Kanten der kleinen Lasche 7 sind gestaltet, dass ein Eindringen der Lasche möglich ist, ohne den Knochen zu sprengen. Da die ganze kleine Lasche im Knochen verankert ist, ist vorzugsweise deren ganze Fläche oberflächenbehandelt.

Die anatomisch geformte grössere Lasche 8 ist der kleinen Lasche 7 gegenüber angeordnet und zwischen 15 - 30° achsversetzt, wie dies am besten aus den Fig. 2 und 3 hervorgeht. Selbstverständlich sind die beiden Laschen den anatomischen Gegebenheiten angepasst. Im Gegensatz zur kleineren Lasche wird die längere nicht eingeschlagen, sondern kommt auf den Beckenknochen zu liegen und wird dort mittels Knochenschrauben 28, Spongiosa- oder Corticalischrauben, befestigt. Intraoperativ bedingt, kann gegebenenfalls die längere Lasche den gegebenen anatomischen Verhältnissen angepasst werden. Einerseits dient diese längere Lasche der Erzielung einer ausreichenden Primärstabilität des Implantates und andererseits der längerfristigen physiologischen Kraftübertragung der auftretenden Belastungen in das Acetabulum. Auch hier sind die Schrauben in den Bohrungen 11 in einem gewissen Bereich schwenkbar, da diese Bohrungen eine sphärische Ansenkung besitzen.

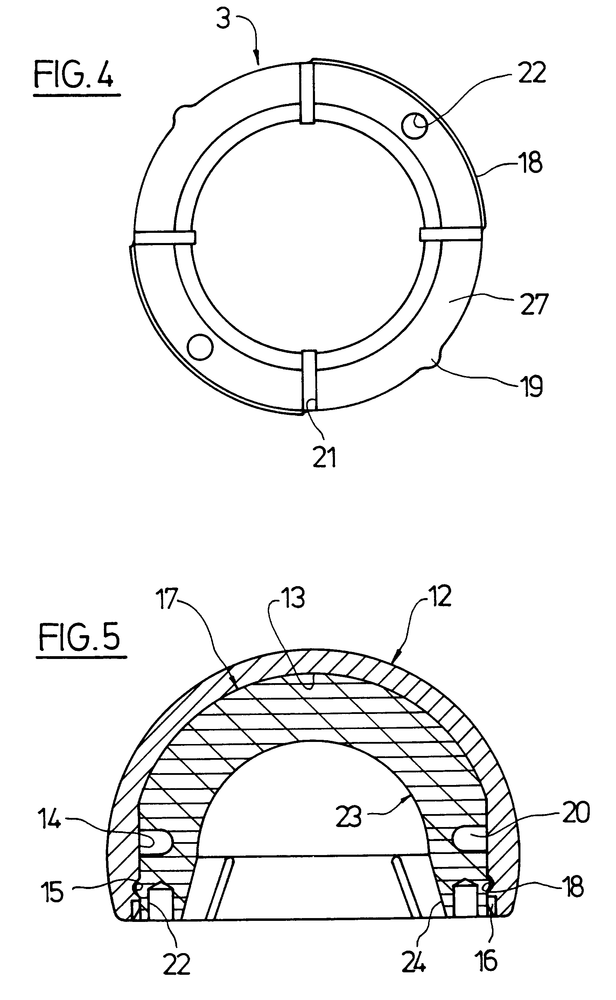

Die Pfannenschale 2 weist aussen eine kugelförmige Oberfläche 12 auf (siehe insbesonder Fig. 5), die den gegebenen anatomischen Verhältnissen am besten angepasst ist, und eine breitflächige, gleichmässige und damit physiologische Kraftübertragung gewährleistet. Auch die ausserhalb des umfassten Bereiches liegende Aussenfläche der Pfannenschale kann mit Mitteln versehen sein, die das Anwachsen des Knochens begünstigen. So kann diese Aussenfläche eine oberflächenvergrössernde Kugelbeschichtung oder eine Beschichtung mit bioaktivem Material wie Hydroxilapatit oder dergleichen aufweisen oder mit einer Aufrauhung versehen sein.

Die Innenfläche 13 der Pfannenschale ist zu einem grossen Teil ebenfalls kugelförmig, weist jedoch zum Aussenrand hin eine zylindrische Fläche 14 auf, in welcher eine umlaufende Nut 15 und, ganz am Rande, Aussparungen 16 angeordnet sind. Die umlaufende Nut 15 sowie die Aussparungen 16 dienen der Befestigung des Einsatzes 3, wie noch weiter unten ausführlicher beschrieben werden wird. Der zylindrische Teil der Pfannenschale übernimmt das Reibmoment, das von der Hüftgelenkkugel auf den Einsatz übertragen wird und verhindert dadurch eine Verschiebung des Einsatzes.

Der Einsatz 3 besteht vorzugsweise aus Polyethylen und dient zur Aufnahme der Hüftgelenkkugel. Die Aussenfläche 17 des Einsatzes entspricht der Innenfläche 13 und 14 der Pfannenschale, wobei der Nut 15 ein Wulst 18 entspricht. Der Wulst 18 ist hier nicht am ganzen Umfang angeordnet sondern nur sektorweise vorhanden. Zur Drehsicherung weist der Einsatz den Aussparungen 16 entsprechende Nocken 19 auf, wobei die Anzahl der Aussparungen bzw. Nocken beliebig sein kann.

Da es vorkommen kann, dass der Einsatz ausgetauscht werden muss, müssen verschiedene Massnahmen dazu vorgesehen sein. So ist es zweckmässig, wenn sich der Wulst nicht über den ganzen Umfang erstreckt, sondern nur über beispielsweise zweimal 90° wie in Fig. 4 angedeutet. Ausserdem muss der Wulst aus der Nut abgehoben werden, falls der Einsatz herausgenommen werden soll, was nur möglich ist, falls der Einsatz zusammenpressbar ist. Dies wird dadurch ermöglicht, dass etwa im inneren Drittel des zylindrischen Teils ein Einschnitt 20 vorgesehen wird und in der Ringfläche 27 des Einsatzes Nuten 21, beispielsweise 4, vorgesehen werden. Durch diese Massnahmen wird eine gewisse Flexibilität im zylindrischen Teil des Einsatzes erreicht und es wird dadurch möglich, mit Hilfe eines Instrumentes, das in zwei Bohrungen 22 eingreifen kann, wobei die Bohrungen dort angebracht sind, wo sich die Wülste 18 befinden, den zylindrischen Teil des Einsatzes zusammenzudrücken.

Die Innenfläche 23 des Einsatzes weist die an sich bekannte Form der Hüftgelenkpfanne auf, mit einer kugelförmigen Fläche, der sich ein erweiterter Teil 24 anschliesst, um eine erhöhte Bewegungsfreiheit zu gewährleisten. Eine mechanische Klemmung, bei Verhinderung von Luxation, des Gelenkkopfes ist möglich, falls die Innenfläche 23 über den Aequator hinausragt.

Der Sicherungsring 4 weist ein selbsthemmendes Aussengewinde 25 auf, das dem Innengewinde des zylindrischen Innenteiles 5 des Stützringes entspricht. Der Innendurchmesser des Sicherungsringes ist leicht grösser als der Aussendurchmesser der Pfannenschale, was zu einer guten Klemmwirkung des Sicherungsringes führt. Um das Einschrauben und Sichern des Sicherungsringes zu ermöglichen, ist die Stirnseite mit Nuten 26 oder Bohrungen oder dergleichen versehen.

Anstatt des vorgehend beschriebenen Klemmringes können auch andere Klemmeinrichtungen verwendet werden, um die Pfannenschale in der ausgerichteten Stellung zu fixieren.

Nach dem Verankern des Stützringes an den vorgesehenen Knochenteilen wird die Pfannenschale eingesetzt und ausgerichtet, um sie in die biomechanisch optimale Stellung zu bringen, wie dies in Figur 2 angedeutet ist, wobei eine Schwenkbarkeit von 30° angenommen wurde, die normalerweise ausreichend ist. Anschliessend wird die Pfannenschale durch den Sicherungsring 4 unverrückbar befestigt. Darauf folgend kann der Einsatz 3 aus Polyethylen in die Pfannenschale eingeführt und eingeschnappt werden. Durch die Möglichkeit, die Pfannenschale in einem weiten Winkelbereich zu schwenken und dadurch optimal zu positionieren und durch das zementlose Befestigen der Pfannenschale im Stützring wird eine sehr gute Langzeitstabilität und eine grösstmögliche Bewegungsfreiheit erzielt.

## Patentansprüche

1. Acetabulum-Pfannenbefestigungsvorrichtung mit einem Befestigungselement (1) mit mindestens einer Befestigungslasche (7, 8) zum Befestigen im und am entsprechenden Knochenteil, einer Pfannenschale (2), die im Befestigungelement (1) schwenkbar orientierbar und mittels eines Sicherungselementes (4) am Befestigungselement (1) befestigbar ist, und einem Einsatz (3) zur Aufnahme des Gelenkkopfes, dadurch gekennzeichnet, dass die Pfannenbefestigungsvorrichtung als Pfannenstützring (1) ausgebildet ist und einen Stützring (1) aufweist, der zu seinem zementlosen Befestigen zwei Befestigungslaschen (7, 8) aufweist und innen ein zylindrisches Teil mit Innengewinde (5) zur Aufnahme des als Ring ausgebildeten Sicherungselementes (4) mit selbsthemmendem Aussengewinde (25) und ein anschliessendes kugelförmiges Teil (6) zur Aufnahme der Pfannenschale (2) aufweist.

2. Pfannenstützring nach Anspruch 1, dadurch gekennzeichnet, dass der Stützring (1) eine kürzere Befestigungslasche (7) mit Löchern (10) zum Befestigen im Schambein und eine längere Befestigungslasche (8) mit Bohrungen (11) zum Befestigen am Beckenknochen aufweist, wobei die Laschen achsversetzt und den Knochenteilen gemäss verschränkt sind.

3. Pfannenstützring nach Anspruch 1 oder 2, dadurch gekennzeichnet, dass die Pfannenschale eine kugelförmige Aussenfläche (12) besitzt und die Innenfläche aus einem zylindrischen Teil (14) und einem kugelförmigen Teil (13) besteht, wobei der zylindrische Teil Mittel (15, 16) zur Sicherung des Einsatzes (3) aufweist und der Einsatz (3) eine der Innenfläche (13, 14) der Pfannenschale (2) entsprechende Aussenfläche (17) mit entsprechenden Sicherungsmitteln (18, 19) aufweist.

4. Pfannenstützring nach einem der Ansprüche 1 bis 3, dadurch gekennzeichnet, dass der Einsatz (3) aus Kunststoff, vorzugsweise Polyethylen, oder zumindest teilweise aus Metall besteht und Mittel (21, 22) zu dessen Herausnahme aus der Pfannenschale aufweist.

5. Pfannenstützring nach Anspruch 3, dadurch gekennzeichnet, dass die Mittel zur Sicherung des Einsatzes in der Pfannenschale aus einer umlaufenden Nut (15) und Ausnehmungen (16) in der Pfannenschale und Wülste (18) sowie Nocken (19) am Einsatz (3) bestehen.

6. Pfannenstützring nach einem der Ansprüche 1 bis 5, dadurch gekennzeichnet, dass der Einsatz aus Polyethylen herausnehmbar ist, wobei der zylindrische Teil aussen einen Einschnitt (20), die Ringfläche (25) Nuten (21) und an Stellen, an denen ein Wulst (18) vorhanden ist, Bohrungen (22) aufweist, um mittels eines in den Bohrungen angreifenden Werkzeugs den Einsatz nahe der Stirnfläche zusammendrücken und von der Pfannenschale abheben zu können.

7. Pfannenstützring nach einem der Ansprüche 1 bis 6, dadurch gekennzeichnet, dass die mit dem Knochen in Berührung kommenden Teile des Stützrings (1) und der Pfannenschale (2) mit Mitteln versehen sind, die das Anwachsen des Knochens begünstigen.

8. Pfannenstützring nach Anspruch 7, dadurch gekennzeichnet, dass die Anwachsmittel aus einer Beschichtung mit bioaktivem Material, insbesondere Hydroxylapatit, Calciumphosphate oder Glas bestehen oder die Aussenfläche aufgerauht ist.

9. Pfannenstützring nach einem der Ansprüche 1 bis 8, dadurch gekennzeichnet, dass der Stützring (1), die Pfannenschale (2) und der Sicherungsring (4) aus Metall, insbesondere eine Chrom-Kobalt-Molybdän-Legierung, Titan oder eine Titanlegierung, oder aus Kunststoff bestehen.

## Claims

1. Acetabular socket fastening device, comprising a fastening element (1) having at least one attaching strap (7, 8) for the attachment in and on the corresponding bone portion, a socket cup (2) which is pivotably orientable in said fastening element (1) and attachable to the fastening element (1) by means of a securing element (4), and an insert (3) intended to receive the joint ball, characterised in that the socket fastening device is in the form of a socket supporting ring (1) comprising a supporting ring (1) which is provided with two attaching straps (7, 8) for its cement-free attachment, as well as an internal cylindrical portion having an internal thread (5) intended to receive said securing element (4) which is in the form of a ring and has a self-locking external thread (25), and a following spherical portion (6) intended to receive the socket cup (2).

2. Socket supporting ring according to claim 1, characterised in that the supporting ring (1) comprises a shorter attaching strap (7) provided with holes (10) for its attachment in the pubis, and a longer attaching strap (8) provided with bores (11) for its attachment to the pelvis, said straps being axially displaced and staggered in accordance with the respective bone portions.

3. Socket supporting ring according to claim 1 or 2, characterised in that said socket cup has a spherical external surface (12) and its internal surface is formed of a cylindrical portion (14) and a spherical portion (13), said cylindrical portion comprising means (15, 16) for the purpose of securing said insert (3), and said insert having an external surface (17) which corresponds to the internal surface (13, 14) of said socket cup (2) and is provided with corresponding securing means (18, 19).

4. Socket supporting ring according to any one of claims 1 to 3, characterised in that said insert (3) is made of plastics material, preferably of polyethylene, or at least partially of metal, and comprises means (21, 22) for its removal from said socket cup.

5. Socket supporting ring according to claim 3, characterised in that said means for securing said insert in said socket cup consist of a circular groove (15) and recesses (16) in said socket cup, and of beads (18) as well as projections (19) on said insert (3).

6. Socket supporting ring according to any one of claims 1 to 5, characterised in that said polyethylene insert is removable, said cylindrical portion having an external incision (20), and said annular surface (25) having grooves (21) and bores (22) at those locations where a bead (18) is provided, in order to allow said insert to be compressed near its front surface and to be lifted off from said socket cup by means of a tool engaging in said bores.

7. Socket supporting ring according to any one of claims 1 to 6, characterised in that the portions of said supporting ring (1) and of said socket cup (2) which are in contact with the bone are provided with means favoring the growth of the bone.

8. Socket supporting ring according to claim 7, characterised in that said growth means consist of a coating of a bioactive material, more particularly hydroxyl apatite, calcium phosphate, or glass, or in that the external surface is roughened.

9. Socket supporting ring according to any one of claims 1 to 8, characterised in that said supporting ring (1), said socket cup (2), and said securing ring (4) are made of metal, more particularly of a chromium-cobalt-molybdenum alloy, of titanium, a titanium alloy, or of plastics material.

## Revendications

1. Dispositif de fixation pour la cavité cotyloïde, comprenant un élément de fixation (1) ayant au moins une languette de fixation (7, 8) pour la fixation dans et à la partie osseuse correspondante, une cupule (2) cavitaire, orientable de manière pivotante dans ledit élément de fixation (1) et pouvant être fixée à l'élément de fixation (1) au moyen d'un élément d'arrêt (4), et une insertion (3) destinée à recevoir la tête d'articulation, caractérisé en ce que ledit dispositif de fixation pour la cavité est réalisé sous forme d'un anneau de soutien (1) cavitaire et présente un anneau de soutien (1) comprenant deux languettes de fixation (7, 8) pour une fixation sans ciment, ainsi qu'une partie cylindrique intérieure avec taraudage (5), destinée à recevoir ledit élément d'arrêt (4) annulaire avec filetage autobloquant (25), suivie d'une partie sphérique (6) destinée à recevoir ladite cupule (2) cavitaire.

2. Anneau de soutien cavitaire selon la revendication 1, caractérisé en ce que l'anneau de soutien (1) comporte une languette de fixation plus courte (7) ayant des alésages (10) pour la fixation dans le pubis, et une languette de fixation plus longue (8) avec alésages (11) pour la fixation au pelvis, les languettes étant désaxées et entrelacées selon les parties osseuses.

3. Anneau de soutien cavitaire selon la revendication 1 ou 2, caractérisé en ce que la cupule cavitaire présente une surface extérieure (12) sphérique et la surface intérieure est constituée d'une partie cylindrique (14) et d'une partie sphérique (13), ladite partie cylindrique présentant des moyens (15, 16) de blocage de l'insertion (3), et l'insertion (3) présentant une surface extérieure (17) adaptée à la surface intérieure (13, 14) de la cupule (2) cavitaire avec des moyens de blocage (18, 19) correspondants.

4. Anneau de soutien cavitaire selon l'une quelconque des revendications 1 à 3, caractérisé en ce que l'insertion (3) est composée de matière synthétique, préférablement de polyéthylène, ou au moins partiellement de métal, et en ce qu'elle présente des moyens (21, 22) pour l'enlever de la cupule cavitaire.

5. Anneau de soutien cavitaire selon la revendication 3, caractérisé en ce que les moyens d'arrêt de l'insertion dans la cupule cavitaire consistent en une rainure circulaire (15) et d'évidements (16) dans la cupule et de bourrelets (18) ainsi que d'ergots (19) à l'insertion (3).

6. Anneau de soutien cavitaire selon l'une quelconque des revendications 1 à 5, caractérisé en ce que l'insertion en polyéthylène est amovible, la partie cylindrique présentant une incision (20) extérieure, et la surface annulaire (25) présentant des rainures (21) et des alésages (22) aux endroits où il y a un bourrelet (18), afin de pouvoir comprimer l'insertion près de la surface frontale au moyen d'un outil engagé dans lesdits alésages et de l'enlever de la cupule cavitaire.

7. Anneau de soutien cavitaire selon l'une quelconque des revendications 1 à 6, caractérisé en ce que les parties de l'anneau de soutien (1) et de la cupule cavitaire (2) en contact avec l os présentent des moyens favorisant l'attachement de l'os.

8. Anneau de soutien cavitaire selon la revendication 7, caractérisé en ce que lesdits moyens d'attachement consistent d'un revêtement en une matière bioactive, plus particulièrement l'hydroxylapatite, le phosphate de calcium ou le verre, ou que la surface extérieure est rugueuse.

9. Anneau de soutien cavitaire selon l'une quelconque des revendications 1 à 8, caractérisé en ce que l'anneau de soutien (1), la cupule cavitaire (2) et l'anneau d'arrêt (4) sont composés de métal, plus particulièrement d'un alliage au chrome-cobalt-molybdène, de titane ou d'un alliage au titane, ou d'une matière synthétique.
